# EUROPÄISCHE PATENTANMELDUNG

(11) **EP 2 011 515 A1**
(43) Veröffentlichungstag der Anmeldung: **07.01.2009**
(21) Anmeldenummer: 07111956.4
(22) Anmeldetag: 06.07.2007
(51) Int. Cl.: A61K 45/06

(54) **Verwendung von Antagonisten von G Protein gekoppelten Rezeptoren (GPCRs)**

(71) Anmelder: E.R.D.E. Diagnostik GmbH, 13125 Berlin (DE)
(72) Erfinder: Die Erfindernennung liegt noch nicht vor
(74) Vertreter: Meyers, Hans-Wilhelm

(57) **Zusammenfassung**

Verwendung von Antagonisten von für G Protein gekoppelte Rezeptoren (GPCRs) zur Herstellung eines Medikaments zur Behandlung und Prophylaxe von durch agonistische Autoantikörper (agAAK) bedingte bzw. mit ihnen assoziierte Erkrankungen.

## Beschreibung

Gegenstand der vorliegenden Erfindung ist die Verwendung von Antagonisten von so genannten G Protein gekoppelten Rezeptoren (GPCRs) zur Herstellung eines Medikaments.

Eine der wichtigsten Krankheitsgruppen beim Menschen sind die sog. Autoimmunerkrankungen. Zu ihnen gehören solche verschiedenen Krankheiten wie Rheumatoide Arthritis, Multiple Sklerose und Diabetes mellitus (Typ1 und Typ2).

Bei Autoimmunkrankheiten richtet sich das Immunsystem gegen körpereigene Strukturen, Zellen und Moleküle. Durch deren Schädigung, die häufig ihren Ausdruck als - lokale - akute oder chronische Entzündung findet, kommt es zu einer Einschränkung oder dem Verlust der betroffenen Zell- und Organfunktion.

Sowohl fehlgeleitete Zellen (z.B. Makrophagen) wie Moleküle (z.B. Antikörper) des Immunsystems, aber auch häufig Produkte von Immunzellen (z.B. proinflammatorische Zytokine) selbst, können autoimmunologische Prozesse induzieren und aufrechterhalten.

Die diagnostische Einstufung einer klinischen Entität als Autoimmunkrankheit ist an eine Reihe von wissenschaftlichen Anforderungen geknüpft. Neben dem diagnostischen Nachweis von z.B. auto-reaktiven T-Zellen oder Autoantikörpern gegen definierte Zielstrukturen wird die Erzeugung des Krankheitsbildes im Tierexperiment allgemein gefordert, ganz im Sinne eines erweiterten Koch'schen Postulats.

Die Therapie von Autoimmunkrankheiten ist fast immer eine Symptomtherapie mit einer mehr oder weniger ausgeprägten antiinflammatorischen Komponente. Bevorzugt werden sog. Immunsuppressiva eingesetzt. Neben nichtsteroidalen antiinflammatorischen Medikamenten sind Substanzen wie Corticosteroide oder gegen proinflammatorische Zytokine gerichtete therapeutische monoklonale Antikörper ("biologics") wirksame Arzneimittel gegen Autoimmunerkrankungen. Sie unterdrücken z.B. die Produktion von autoreaktiven Antikörpern, hemmen die Proliferation von Leukozyten oder blockieren die Wirkung von proinflammatorischen Zytokinen.

Aus tierexperimentellen Untersuchungen ist bekannt, dass Autoantikörper lange Zeit vor der Ausbildung von klinischen Symptomen auftreten können. Die Behandlung solcher Autoimmunkrankheiten hat nicht nur den üblichen indikativen Aspekt sondern auch eine prophylaktische Komponente zur Verhinderung des Auftretens von klinischen Symptomen der Erkrankung.

Ein bekanntes klassisches Beispiel ist die Behandlung der HIV-Infektion. Bereits vor dem Auftreten von klinischen Symptomen wird an Hand von Labordaten wie Anzahl der Viren im Blut oder Anzahl von bestimmten Lymphoztenpopulationen eine virostatische Therapie eingeleitet; im Prinzip prophylaktisch um den Ausbruch der eigentlichen Krankheit zu verhindern oder zumindest herauszuzögern. Ein weiteres Beispiel ist die Behandlung von schwerer Rheumatoider Arthritis mit therapeutischen monoklonalen Antikörpern gegen den Tumornekrosefaktor α.

Eine Sonderstellung unter den Autoimmunerkrankungen nehmen Erkrankungen ein, die mit Autoantikörpern gegen extrazelluläre Schleifen von G Protein gekoppelten Rezeptoren (GPCRs) assoziiert sind (Übersicht bei Wallukat G. *et al.,* in: Brinckmann R and Kunze R, 2002). Zu ihnen gehören insbesondere die kardio- und nephrovaskulär relevanten β1-, β2-Rezeptoren (β1-R, β2-R), α-Rezeptoren (α-R) und AngiotensinII-Rezeptoren Typ 1 (AT1-R). Sie kommen bei so unterschiedlichen Erkrankungen wie Dilatativer Cardiomyopathie (DCM, β1-R), primärem Offenwinkelglaukom (POWG, β2-R) sowie Präeklampsie, vaskulär-nekrotischer Nierentransplantatabstoßung (AT1-R), Hypertonie (α-R, AT1-R) und Diabetes 2 (α-R, β1-R, AT1-R) vor.

GPCRs sind eine weit verbreitete Gruppe von Rezeptoren. Ihre Aminosäurekette durchquert 7x die Zellmembran. Intrazellulär an das namensgebende G Protein gekoppelt, sind sie für Zellen essentiell. Über sie regeln sie den Signaltransport, wenn z.B. Hormone extrazellulär an sie binden. So z.B. wird die Kontraktion glatter, quergestreifter und der Herzmuskelzellen über sie gesteuert oder mitgesteuert.

Seit einiger Zeit ist bekannt, dass Autoantikörper gegen GPCRs diese Rezeptoren ebenso wie die physiologischen Agonisten aktivieren können *(*Literaturübersicht in Brinckmann R and Kunze R (edts.), 2002). Übertragen auf die *in vivo*-Situation führt die antikörperbedingte Zellaktivierung zu einer Dauerbelastung des Organs, z.B. des Herzmuskels, mit den Folgen eines Verlustes an Herzleistung und einem sog. remodelling des Herzmuskelgewebes.

Für DCM wurde die Kausalität der Antikörper gegen den β1-R bei der Entstehung und Aufrechterhaltung dieser Herzkrankheit im Tierexperiment gezeigt (Jahns R *et al.,* 2004; Matsui S *et al.,* 1997; Matsui S *et al.,* 2006). Die Antikörper lassen sich in Tieren durch die Immunisierung mit den antigenen Epitopen aus dem β1-R induzieren, und die Tiere erkranken nach ca. 9-12 Monaten an einer Herzerweiterung, die der menschlichen DCM sehr ähnlich ist. Werden die Immunglobuline der antikörperpositiven Tiere in gesunde Tiere infundiert, erkranken diese ebenfalls nach ca. 9-12 Monaten, d.h. diese Zeit vergeht, bevor die pathophysiologische Wirkung der transferierten Autoantikörper zu einem klinischen Bild führt.

Die Arbeitsgruppe um Matsui (2006) zeigte kürzlich in einem Experiment an Kaninchen, dass die spezifische Entfernung der gegen den β1-R gebildeten Antikörper mittels einer extrakorporalen Immunadsorption zu einer Rückbildung der antikörperbedingten linksventrikulären Herzdilatation führt. Dies bestätigt die am Menschen mittels einer spezifischen Immunadsorption erreichten klinischen Verbesserung bei Patienten mit DCM und agAAK gegen den β1-R (Wallukat G *et al.,* 2002).

Die pathophysiologische Bedeutung von agAAK gegen den AT1-R wurde ebenfalls tierexperimentell belegt. In einem Transplantationsmodell an der Ratte sind humane agAAK, die aus Patienten mit vaskulär-nekrotischer Nierentransplantatabstoßung isoliert wurden, pathologisch aktiv am AT1-R und induzieren in der transplantierten Niere eine histologisch sichtbare Gefäßnekrose (Dragun D *et al.,* 2005).

Die therapeutische Wirkung der Rezeptorantagonisten ("Betablocker", AT1-Rezeptorantagonisten) bei Indikationen wie chronische Herzinsuffizienz, DCM oder Bluthochdruck beruht auf einer Aufhebung oder Reduktion der Wirkung der körpereigenen Rezeptoragonisten. Im Fall des β1-R wird die Wirkung von Adrenalin/Noradrenalin gedämpft mit der Folge, dass der Herzmuskel seine Leistung reduziert. AT1-Rezeptor-Antagonisten senken u.a. den Blutdruck, indem sie die Bindungsstellen von Angiotensin an den korrespondierenden Rezeptoren blockieren.

Während im Blut die physiologischen Agonisten eine biologische Halbwertszeit von wenigen Minuten haben, ist diese bei Immunglobulinen deutlich länger. Für die Immunglobuline IgG1, IgG2 und IgG4 beträgt sie 21 Tage, für IgG3 ca. 7 Tage. Dies dürfte eine der wesentlichen Ursachen sein für die durch agAAK bzw. die langandauernde Rezeptoraktivierung ausgelösten pathophysiologischen Veränderungen an Geweben und Organen.

In einem zellulären Testsystem mit neonatalen Rattenkardiomyozyten wirken z.B. Antikörper gegen den β1-R oder AT1-R ähnlich wie die korrespondierenden Hormone Adrenalin und Angiotensin, d.h. sie erhöhen die spontane Pulsationsrate der Rattenkardiomyozyten. Demgemäß spricht man auch von agonistischen Autoantikörpern (agAAK), da sie funktionell den Agonisten ähnlich sind.

Die pathophysiologische Bedeutung der agAAK scheint sich aus ihrer Eigenschaft der Dauerstimulation von Zellen via der Antikörperbindung an Rezeptoren zu ergeben. Mit der Antikörperbindung verbunden ist der Verlust der Fähigkeit der Rezeptordesensibilisierung (Wallukat *et al.,* 1991), d.h. die Zellen können sich nicht mehr mit den ihnen gegebenen Regelkreisen vor der antikörpervermittelten Überaktivierung schützen.

Auf molekularer Ebene beruht der agonistische Effekt der Autoantikörper auf ihrer Fähigkeit zur Rezeptorhomodimerisierung, die zur Rezeptor- bzw. Zellaktivierung führt. Dabei bindet je eine antigene Bindungsstelle vom IgG an je ein extrazelluläres Epitop auf den extrazellulären Schleifen (auf Loop 1 oder Loop 2). Die Antikörperbindung ist sehr stabil und lässt sich im physiologischen Milieu durch Waschprozesse nicht aufheben (Wallukat G, öffentlicher Vortrag am 22.02.2007; Hörsaal der Augenklinik der Universität Erlangen).

Es wurde beschrieben, dass der durch die Antikörperbindung an die zuvor genannten Rezeptoren (Ausnahme: Endothelinrezeptor A, muskarinerger M2-Rezeptor) in vitro auf neonatalen Rattenkardiomyozyten über mehrere Stunden anhält (Wallukat *et al.,* 1991). Rezeptorspezifische Antagonisten vermögen - in vitro an spontan pulsierenden neonatalen Rattenkardiomyozyten - den positiv chronotropen Effekt aufzuheben, d.h. die Zugabe eines Antagonisten, korrespondierend zum Antikörper/Rezeptor, führt zu einer Deaktivierung des Rezeptors. Diese wird sichtbar durch die Reduktion der antikörperinduzierten Steigerung der Pulsationsrate der Rattenkardiomyozyten. Der Prozess ist spezifisch, und ein Antagonist reduziert nur dann die Pulsationsrate, wenn der zuvor eingesetzte Antikörper spezifisch für denselben Rezeptor ist.

Die vorliegende Erfindung hat sich die Aufgabe gestellt, Personen, die an Erkrankungen wie Diabetes Typ2, einer kardiovaskulären, nephrologischen, urologischen, opthalmologischen oder neurologischen Erkrankung leiden und gleichzeitig agAAK gegen GPCRs wie dem Angiotensin II Rezeptor 1, den Alpha-Rezeptoren oder den β1/β2-Rezeptoren tragen, therapeutisch zur Reduktion von autoantikörpervermittelten Symptomen oder vorbeugend zu behandeln.

Gelöst wird die Aufgabe erfindungsgemäß durch eine Verwendung von Antagonisten von so genannten G Protein gekoppelten Rezeptoren (GPCRs) zur Herstellung eines Medikaments zur Behandlung von durch agonistische Autoantikörper (agAAK) bedingten bzw. mit ihnen assoziierten Erkrankungen.

Gegenstand der Erfindung ist gleichermaßen die Verwendung von Rezeptorantagonisten nicht nur zur Dämpfung der Wirkung physiologischer Agonisten, sondern auch zur Aufhebung der Bindung von Autoantikörpern an GPCRs durch Ablösung der agAAK von Rezeptoren sowie die Blockade des Bindens der agAAK an Rezeptoren.

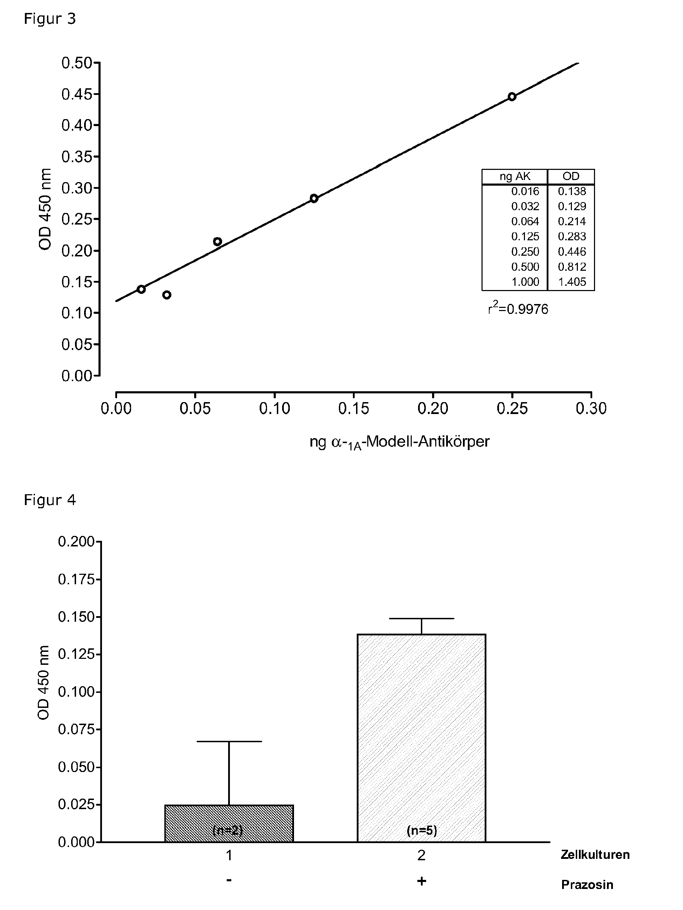

Die antagonistische Wirkung der Rezeptorantagonisten auf die agonistische Wirkung der Autoantikörper wird überraschenderweise begleitet von einer Ablösung der Autoantikörper aus ihrer Bindung an die Rezeptoren (s. Fig. 3).

Die vorliegende Erfindung erweitert die Therapie von Erkrankungen durch die Verwendung von Rezeptorantagonisten zur Aufhebung und Verhinderung antikörpervermittelter pathologischer Zellaktivierungen. Rezeptorantagonisten können erfindungsgemäß bei Patienten zu therapeutischen Zwecken eingesetzt werden, bei denen im Blut agAAK nachgewiesen wurden. Es sollte berücksichtigt werden, dass der jeweilige Rezeptorantagonist für den GPCR indiziert ist, gegen den agAAK nachgewiesen wurden. An lebenden Zellen wurde gefunden, dass an Rezeptoren spezifisch gebundene agAAK durch die Zugabe von entsprechenden Antagonisten (bei agAAK gegen den β1-Adrenozeptor ein sog. Betablocker, bei agAAK gegen den α1-Adrenozeptor ein sog. Alphablocker usw.) sich der agAAK aus seiner Bindung an den Rezeptor ablöst. Die Ablösung wird dabei nachgewiesen im Zellkulturüberstand, der die Immunglobuline enthält, die in einem zweiten Testsystem, einem sensiblen Enzymimmunoassay, antigenspezifisch nachgewiesen werden. Beispielhaft wird dies gezeigt für einen agonistischen Antikörper gegen den humanen α1A-Adrenozeptor, seinen spezifischen Antagonisten Prazosin unter Verwendung von biologischen Testsystemen.

Die erfindungsgemäße Verwendung betrifft GPCRs, die insbesondere ausgewählt sind aus der Gruppe bestehend aus β1-R, β2-R, AT1-Rezeptor, α1-Rezeptoren sowie dem Endothelinrezeptor (bevorzugt Subtyp 1A). Soweit bei den mit Autoantikörpern gegen o.g. GPCRs Prävalenzen für die agAAK bekannt sind, sind sie in Tab.1 dargestellt. Die erfindungsgemäße Verwendung betrifft weiterhin Erkrankungen wie Arteriosklerose, Diabetes 2, Patienten mit terminaler Niereninsuffizienz verschiedene Formen von Demenzen, insbes. Morbus Alzheimer, verschiedene Formen des Glaukoms (Normaldruckglaukom, Pseudoxfoliantes Glaukom) sowie Prostatahyperplasie, erektile Dysfunktion, Tinnitus, Migräne, Dauerkopfschmerz, periphere Durchblutungsstörungen soweit sie mit agAAK gegen o.g. GPCRs assoziiert sind.

**Tab. 1: Agonistische Autoantikörper gegen G Protein gekoppelte Rezeptoren bei verschiedenen Krankheiten (modifizierte Tab. aus: Brinckmann R und Kunze R edts., 2002; die verwendeten Publikationen zur Darstellung der Prävalenzdaten sind in der Originalpublikation enthalten)**

| **Rezeptor** | **Krankheit** | **Prävalenz** |
|---|---|---|
| α_{1A}-R | Hypertonie | 44 % |
| β₁-R | Dilatative Cardiomyopathie | 80 % |
| β₁-R | Peripartum Cardiomyopathie | 100 % |
| β₁-R | Myocarditis | 80 % |
| β₁-R | Chagas' Disease | 29 % |
| β₂-R | Chagas' Disease | 12 % |
| β₂-R | Primäres Offenwinkelglaukom | ca.80 % |
| AT1-R | Preeclampsie | 100 % |
| AT1-R | maligne Hypertonie | 14-33 % |
| AT1-R | vasculär-nekrotische Nierentransplantabstossung | 100 % |
| muskarinerger R (M₂) | Chagas' Disease | 77 % |
| muskarinerger R (M₂) | Dilatative Cardiomyopathie | 30-40 % |
| muskarinerger R (M₂) | idiopathische atriale Fibrillation | 23% |
| Glutamatrezeptor | Rasmussen's Encephalitis, nicht | nicht bekannt |
| (GluR3) | entzündliche fokale Epilepsie | |
| TSH-R | Grave's disease | > 95 % |

| | | |
|---|---|---|
| α-R - α-adrenerger Rezeptor, β-R - β-adrenerger Rezeptor, AT1-R - Angiotensin-II Rezeptor 1, TSH-R - Thyroidhormonrezeptor | | |

Die erfindungsgemäß verwendbaren Antagonisten sind insbesondere ausgewählt aus der Gruppe bestehend aus Antagonisten gegen die o.g. GPCRs. Tab2. gibt beispielhaft einen Überblick.

**Tab. 2: Klinisch relevante GPCRs und therapeutisch verfügbare Rezeptorantagonisten**

| **Rezeptor** | **Antagonisten (Beispiele)** | **Indikationen** |
|---|---|---|
| α1-R | Phenoxybenzamin, Prazosin, Doxazosin, Terazosin, Alfuzosin, Tamsulosin, Bunazosin | Prostatahyperplasie, Tinnitus, Bluthochdruck |
| β1-R | Atenolol, Metoprolol, Bisoprolol, Propranolol, Sotalol, Pindolol, Nevibolol, Carvedilol, | Chronische Herzinsuffizienz, Dilatative Kardiomyopathie, Bluthochdruck, Tachykardien, koronare Herzkrankheit, Migräne |
| β2-R | Timolol (topische Anwendung) | Glaukom |
| AT1-R | Valsartan, Losartan, Irbesartan, Candesartan, Eprosartan, Telmisartan, Olmesartan | Bluthochdruck, akuter Herzinfarkt, Nierenschädigung |
| muskarinerger R (M2) | Atropin (topisch) | Ophthalmologie (Diagnostik) |
| Endothelinrezeptor (ET1A) | Bosentan, Darusentan | pulmonale arterielle Hypertonie, chronische Herzinsuffizienz |

| | | |
|---|---|---|
| α1-R - α1-adrenerger Rezeptor, β-R - β-adrenerger Rezeptor, AT1-R - Angiotensin-II Rezeptor 1 | | |

Die erfindungsgemäße Verwendung der Antagonisten umfasst auch deren Kombination mit anderen Wirkstoffen, z.B. solchen zur Behandlung des akuten Myokardinfarkts, der chronischen Herzinsuffizienz, der Niereninsuffizienz, und des Bluthochdrucks, (s.Tab.1). Beispiele hierfür sind sog. ACE-Hemmer, Herzglykoside, Antiarrhythmika, Diuretika sowie entzündungshemmende oder durchblutungsfördernde Mittel.

So wie die Kardiomyozyten durch physiologische Rezeptoragonisten stimuliert werden, so werden sie auch durch die agAAK stimuliert, und in beiden Fällen wird die Stimulation durch die entsprechenden Rezeptorantagonisten aufgehoben. Aus klinischer Sicht ist die Reduktion der Wirkung der agAAK mit der klassischer Immunsuppressiva zu vergleichen. Es ist allerdings nicht offenbar, wodurch die Wirkung der agAAK aufgehoben wird, d.h. es ist nicht bekannt, ob die Wiederherstellung der Zellfunktion auf einer Änderung der zellulären Rezeptorausstattung beruht oder andere Ursachen hat. Aus immunologischer Sicht handelt es sich dabei nicht um eine immunsuppressive Therapie, sondern um ein neuartiges pharmakologisches Wirkprinzip, welches die Wirkung der agAAK an ihrem Target, dem entsprechenden GPCR, aufhebt.

Immunsuppressive Arzneimitteltherapien werden bei Krankheiten mit nachgewiesenen, pathologisch funktionell relevanten Autoantikörpern durchgeführt. Ihr Ziel ist die Blockade, Neutralisation oder zumindest Reduktion von pathologischen und klinisch wirksamen Autoimmunmechanismen. In der Therapie gegen agAAK sind diese Immunsuppressiva klinisch nicht wirksam. Erfindungsgemäß kann diese Aufgabe aber durch Antagonisten gegen GPCRs wahrgenommen werden, die die Bindung der agAAK an die Rezeptoren aufheben und die agAAK von ihren Rezeptoren ablösen.

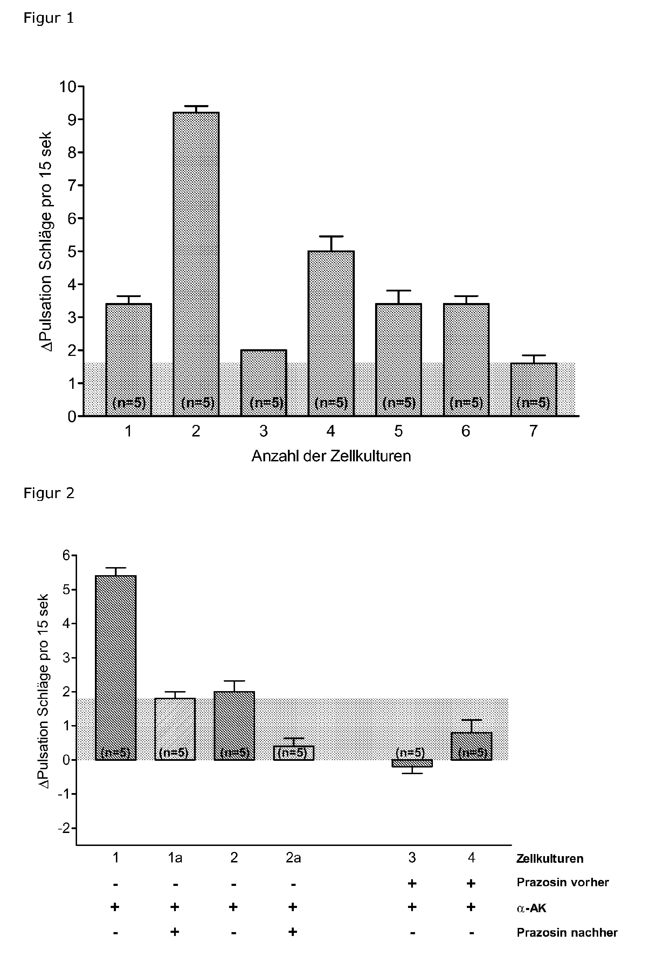

Die in Fig. 2, 3 und 4 gezeigten Daten belegen beispielhaft die Ablösung von agAAK die gegen den humanen α1A-Adrenozeptor gerichtet sind. Für die Experimente wurde ein im Kaninchen generierter Modellantikörper vom IgG Typ benutzt. Als Antigen diente die Aminosäuresequenz des vollständigen extrazellulären 2. Loops des genannten Rezeptors mit der Aminosäuresequenz ¹⁶⁸PAPEDETICQINEE¹⁸¹, in welcher das Bindungsepitop der humanen Autoantikörper enthalten ist. Der Modellantikörper hat identische agonistische Rezeptor aktivierende Eigenschaften wie der bei Menschen mit Bluthochdruck vorkommende agonistische Autoantikörper.

Nach der Zugabe des Modellantikörpers erhöht sich erwartungsgemäß die Pulsationsrate der Rattenkardiomyozyten (Fig.1). Die experimentelle Vorgehensweise zur beispielhaften Demonstration der Eigenschaft der Rezeptorantagonisten agonistische Antikörper vom Rezeptor abzulösen, ist in den Legenden der Fig. 2, 4 und 5 beschrieben. Diesen Ergebnissen zufolge beruht die antagonistisch vermittelte Deaktivierung der durch agAAK verursachten Steigerung der Pulsationsrate auf einer Dissoziation der agAAK von ihren Bindungsorten am GPCR.

Ein weiterer Teil der Erfindung betrifft die Verwendung von Antagonisten gegen GPCRs zur Verhinderung einer Antikörperbindung am Rezeptor. Behandelt man neonatale Rattenkardiomyozyten mit einem Antagonisten vor, bleibt die stimulierende Wirkung danach applizierter agAAK des korrespondierenden Rezeptors aus. Der zeitversetzt nach Prazosin zugesetzte Antikörper kann demnach seine agonistische Wirkung gegenüber den Zellen nicht entfalten. Beispielhaft ist dies in Fig. 2 (Säulen 3 und 4) dargestellt. Diese Eigenschaft der Antagonisten, die Bindung von agAAK zu verhindern, ist überraschend, da die agAAK hochspezifisch an definierte Regionen (Epitope) auf den extrazellulären Schleifen 1 oder 2 der jeweiligen GPCRs binden, also in Regionen, die nicht identisch sind mit dem Bindungsort der Antagonisten.

Die durch Antagonisten am Rezeptor ausgelöste Konformationsänderung blockiert demnach nicht nur die Wirkung der korrespondieren Agonisten sondern auch die der Autoantikörper und schützt damit den Rezeptor vor einer pathologischen autoimmunologisch bedingten Aktivierung.

Die überraschenden Befunde zur Dissoziation des Antikörpers vom Rezeptor wurden an neonatalen Rattenkardiomyozyten erhoben.

### Methode und Materialien zur Bestimmung agonistischer Autoantikörper im Bioassay

Die Bestimmung agAAK erfolgt funktionell in einem Bioassay unter Verwendung neonataler Rattenkardiomyozyten.

Zur Bestimmung der agAAK in Patientenseren (ohne Antikoagulation) werden aus diesen die Immunglobuline präpariert wie beschrieben bei Wallukat *et al.* (1991). In einer Endverdünnung von 1:20 bis 1:100 werden die Proben dann in die Zellkulturflaschen den spontan pulsierenden Rattenkardiomyozyten zugesetzt.

Entsprechend ihrer Ausstattung an GPCRs ist die Identifikation von agAAK gegen 6 verschiedene Rezeptortypen mit dem Bioassay möglich, im einzelnen der α1-R, der β1-R, der β2-R, der AT1-R, der Endothelinrezeptor (Subtyp 1A) und der muskarinerge Rezeptor M2. Auf Grund einer sehr hohen, wenn nicht identischen Sequenzhomologie dieser GPCRs bei Mensch und Ratte ist die Bindung der humanen Autoantikörper an den extrazellulären Rezeptorschleifen der Rattenkardiomyozyten möglich. Mit diesem Funktionstest können nicht nur humane agAAK identifiziert und charakterisiert werden. Im Tier durch Immunisierung mit Rezeptorpeptiden generierte Antikörper sind ebenfalls agonistisch aktiv und nachweisbar. Die Erfindung wird daher beispielhaft dargestellt durch die Verwendung eines im Kaninchen generierten und affinitätschromatografisch gereinigten Immunglobulins (IgG), welches ebenfalls agonistisch wirkt und dieselben Epitope auf den Rezeptorschleifen erkennt wie seine humane, krankheitsassoziierte Entsprechung.

Die Präparation der neonatalen Rattenkardiomyozyten und das Anlegen der Zellkultur erfolgt in Anlehnung an Wallukat G und Wollenberger A (1987) und Wallukat *et al.* (1991). Spontan pulsierende neonatale Rattenherzzellen werden am 4. bis 6. Tag der Kultivierung in den Bioassay eingesetzt. Die Bestimmung der agAAK erfolgt in drei Blöcken (Abstand 20 min) á 5 Zellkulturflaschen (12,5 cm² Oberfläche, Fa. BD Falcon). In Vorbereitung der Zählung eines Durchgangs wird in maximal 15 Zellkulturflaschen das Kulturmedium (stets 2 ml) gewechselt, und die Flaschen werden 1 Stunde bei 37°C stationär kultiviert. 5 min vor Beginn der Zählung werden die ersten 5 Flaschen kurzzeitig geöffnet und auf die Heiztisch (37°C) eines Mikroskops gestellt.

Nacheinander wird in diesen fünf Flaschen die basale Pulsationsrate der Zellen bestimmt. Dabei befindet sich die zu zählende Flasche in einer Metallschablone mit Zählfeldern. In mindestens 5 ausgewählten Zählfeldern dieser Schablone wird die Pulsationsrate der Zellen Computer-gestützt bestimmt. Anschließend werden die aus Patientenserum isolierten Immunglobuline (IgG) in einer Verdünnung von 1:20 bzw. 1:40 zugesetzt. Werden Modellantikörper verwendet, werden diese gelöst in phosphatgepufferter physiologischer Kochsalzlösung in einer Endkonzentration von 2,5 µg/ml eingesetzt.

Nach einstündiger Inkubation stationär bei 37°C werden die Zellkulturflaschen wiederum für 5 min auf die Heiztisch (37°C) des Mikroskops gestellt und die Pulsationsrate der Zellen in den gleichen mindestens 5 Zählfeldern erneut Computer-gestützt bestimmt.

AgAAK positive Proben führen bei den Rattenherzzellen in aller Regel zu einer Erhöhung der Pulsationsrate durch die zugegebenen Immunglobuline um mehr als 2 Schläge pro 15 sek. Die anschließende, zusätzliche Inkubation der Zellkulturen mit Antagonisten und der darauf folgende Rückgang der Pulsationsrate in Richtung basale Pulsationsrate bestätigt die Existenz agAAK in dem Patientenserum.

Die Spezifität der agAAK für einen Rezeptortyp wird über die Zugabe von Antagonisten in die Zellkultur ermittelt. Im selben Testsystem kann bei Bedarf ohne Zusatz von Antagonisten durch eine Vorinkubation der zu prüfenden Immunglobulinfraktion (bzw. mit den darin enthaltenen Autoantikörpern) mit den langkettigen extrazellulären Loop-Peptiden (vollständige Aminosäuresequenz) bzw. den kurzkettigen Peptiden (ca. 7 Aminosäuren, enthalten u.U. Antikörperbindungsstelle = Epitop) die Rezeptorspezifität der Autoantikörper ermittelt werden. Bleibt nach Vorinkubation im Bioassay das positiv chronotrope Signal bei einem bestimmten Peptid aus, ist dies ein Beweis für das Vorliegen eines agAAK, der sich gegen den GPCR richtet, aus dessen Aminosäuresequenz (vorzugsweise extrazelluläre Loops 1 oder 2) er stammt (Wallukat *et al.,* 1999).

Die Zählung der Pulsationsrate erfolgt mit folgenden technischen Mitteln:
- Olympus Mikroskop, Modell IMT-2, mit Heiztisch, *Olympus optical co., LTD., Japan*
- Zeiss Mikroskop, Typ TELAVAL3, mit Heiztisch, *Carl-Zeiss-Jena, Deutschland*
- Metallschablone mit definierten Zählfeldern
- Programm-Software: Imagoquant Lab View 5.1, *Mediquant GmbH, Deutschland*

Die eingesetzten Chemikalien waren vom Reinheitsgrad p.A. und stammten von der Fa. Sigma. Zellkulturmedien, Zellkulturflaschen und weitere Materialen wurden von verschiedenen bekannten Herstellern bezogen.

### Methode und Materialien zur Messung der Antikörper im Enzymimmunoassay

Der verwendete Antikörper lässt sich als Molekülmasse in einem sensitiven Enzymimmunoassay bestimmen (Fig.3). Als Antigen wurde zu diesem Zweck ein Peptid, das dem extrazellulären Loop 2 des humanen α1A-Adrenozeptors entspricht, über Biotin mit einem sog. PEG (Polyethylenglycol)-Spacer am N-terminalen Ende der Aminosäurekette gekoppelt.

Zwecks Prüfung, ob die Zugabe des Rezeptorantagonisten zu einer Freisetzung der an die Rezeptoren gebundenen Antikörper führt, wurde der Zellkulturüberstand abgenommen und in einem spezifischen Enzymimmunoassay mit dem extrazellulären Loop 2 als Antigen quantitativ nachgewiesen.

Nach einstündiger Koinkubation des Loop-Peptids (100 ng/ml) mit dem Kaninchenantikörper in unterschiedlichen Konzentrationen wurde der Immunkomplex auf mit Neutravidin beschichtete Mikrotiterplatten (Fa. *Pierce)* gegeben. Dies führt zur Bindung des biotinylierten Peptids an die Plattenoberfläche via Neutravidin. Anschließend wurden die Kavitäten gewaschen (jeweils 200µl, 3 mal mit 25 mM Tris gepufferter 0,15 M Kochsalzlösung enthaltend 0,05% Tween 20, pH 7.2, Fa. *Pierce).* Nach Zugabe eines sekundären Antikörpers (30 min, Anti-Kaninchen-IgG, Peroxidasekonjugiert, 1:100.000, *Dianova)* und Zugabe des TMB Substrates (15 min, *Pierce)* wurde das umgesetzte Enzymsubstrat anschließend photometrisch bei 450 nm gemessen.

### Legenden zu den Figuren

**Figur 1****:** Steigerung der spontanen Pulsationsrate neonataler Rattenkardiomyozyten durch einen im Kaninchen generierten agonistischen Antikörper (IgG) gegen den 2. extrazellulären Loop des α1A Adrenozeptors.

Es wurde der Modellantikörper (Endkonzentration 2,5 µg/ml) der Zellkultur zugesetzt. Die Pulsationsraten der Zellen wurden vor und 60 min nach Zugabe des Modellantikörpers bestimmt. Dargestellt sind die Daten von 7 Parallelkulturen als Differenz zu den sog. Basalraten und pro 15 Sekunden. Pro Zellkulturflasche wurde die Pulsation von Zellen aus 5 Zellpopulationen gemessen (s. Zahl in der jeweiligen Säule). Dargestellt wurde der Mittelwert sowie SEM (standard error of the mean). Bei Säule 3 ist die Fehlerbreite extrem klein und daher nicht sichtbar. Eine Steigerung der Pulsationsrate < 1,7 Schläge/15 sec wird noch nicht als Nachweis eines agAAK gewertet. Sie ist als heller Graubereich zwischen den Säulen gekennzeichnet. Die Fig.1 zeigt typische Beispiele einer Antikörper-induzierten Steigerung der Pulsationsrate von verschiedenen Zellkulturen.

### Figur 2: Einfluss des rezeptorspezifischen Antagonisten Prazosin auf die Bindung des Antikörpers an den α1A-Adrenozeptor

Den in Fig.1 dargestellten Zellkulturen wurde nach 1 h Inkubation mit dem Antikörper und der Messung der Pulsationsrate der Rezeptorantagonist Prazosin (10-7M) zugesetzt (Grafiksäulen 1 und in der Wiederholung 2). Das - erwartete, weil aus der Literatur bekannte - Ergebnis ist die Reduktion der Pulsationsrate der Zellen. Diese ist dargestellt in den mit jeweils 1a und 2a bezeichneten Säulen. Die Messung erfolgte 10 min nach Zusatz von Prazosin,

In parallelen Zellkulturen wurde der umgekehrte Weg beschritten, d.h. die Zellen wurden zunächst mit dem Rezeptorantagonisten Prazosin vorinkubiert (10 min). Anschließend wurde der Antikörper zugesetzt und 1 h inkubiert. Wie aus den Grafiksäulen 3 und 4 (Wiederholung von 3) ersichtlich, blockiert der Antagonist Prazosin die Induktion der antikörpervermittelten Steigerung der Pulsationsrate. Unter der Abszisse die jeweiligen Zusätze genannt.

### Figur 3: Bindung des Modellantikörpers gegen den 2. extrazellulären Loop des humanen α1A-Adrenozeptors im Enzymimmunoassay (EIA).

Der quantitative Nachweis des gegen den 2. extrazellulären Loop gerichteten Antikörpers gelingt in einem EIA. Fig. 3 zeigt eine typische, lineare Eichkurve.

### Fig. 4: Ablösung des Antikörpers von spontan pulsierenden Rattenkardiomyozyten durch den Antagonisten Prazosin

Zur Demonstration der Antikörperablösung wurden die Zellkulturen aus Fig.1 verwendet. Nach 1h Inkubation wurde der nicht gebundene Antikörper ausgewaschen. Anschließend wurde den Kulturen 3-7 Prazosin zugesetzt und die Zellkultur 20 min inkubiert. Danach wurden Zellkulturüberstände aus allen Kulturen entnommen und im EIA die Antikörpermenge bestimmt; Säule 1 (n=2) ohne Prazosin, Säule 2 (n=5) mit Prazosin. Die Daten zeigen, dass Prazosin den Antikörper aus der Bindung löst, was sich in einer höheren Antikörperkonzentration in den Zellkulturüberständen der Kulturen 3-7 (Säule 2) darstellt.

### Literaturverzeichnis

1. Brinckmann R and Kunze R (edts.). G-protein coupled receptors and autoantibodies, 2002 Pabst Science Publishers, ISBN 3-936142-93-9.
2. Dragun D, Müller DN, Brasen JH, Fritsche L, Nieminen-Kelha M, Dechend R, Kintscher U, Rudolph B, Hoebeke J, Eckert D, Mazak I, Plehm R, Schonemann C, Unger T, Budde K, Neumayer HH, Luft FC, Wallukat G (2005). Angiotensin II type 1-receptor activating antibodies in renal-allograft rejection. N Engl J Med., 352(6):558-569.
3. Jahns R, Boivin V, Hein L, Triebel S, Angermann CE, Ertl G, Lohse MJ. (2004). Direct evidence for a beta 1-adrenergic receptor-directed autoimmune attack as a cause of idiopathic dilated cardiomyopathy. J Clin Invest. 113(10):1419-1429.
4. Matsui S, Fu ML, Katsuda S, Hayase M, Yamaguchi N, Teraoka K, Kurihara T, Takekoshi N, Murakami E, Hoebeke J, Hjalmarson A (1997). Peptides derived from cardiovascular G-protein-coupled receptors induce morphological cardiomyopathic changes in immunized rabbits. J Mol Cell Cardiol. 29(2): 641-655.
5. Matsui S, Fu M, Hayase M, Katsuda S, Yamaguchi N, Teraoka K, Kurihara T, Murano H, Takekoshi N (2006). Transfer of immune components from rabbit autoimmune cardiomyopathy into severe combined immunodeficiency (SCID) mice induces cardiomyopathic changes. Autoimmunity, 39(2): 121-128.
6. Wallukat G und Wollenberger A (1987). Involvement of beta2-adrenergic receptors in the potentiation of the chronotropic action of isoprenaline evoked in rocker-cultured neonatal rat heart cells by pyruvate and L(+)-lactate. In: Beamish RE, Panagia V, Dhalla NS; Eds. Pharmacological aspects of heart diseases. Boston: Martinus Nijhoff, p 217-231.
7. Wallukat G, Morwinski M, Kowal K, Forster A, Boewer V, Wollenberger A (1991). Autoantibodies against the beta-adrenergic receptor in human myocarditis and dilated cardiomyopathy: beta-adrenergic agonism without desensitization. Eur Heart J.; 12 Suppl D: 178-181.
8. Wallukat G, Homuth V, Fischer T, Lindschau C, Horstkamp B, Jupner A, Baur E, Nissen E, Vetter K, Neichel D, Dudenhausen JW, Haller H, Luft FC (1999). Patients with preeclampsia develop agonistic autoantibodies against the angiotensin AT1 receptor. J Clin Invest.; 103(7): 945-952.

## Patentansprüche

1. Verwendung von Antagonisten von sogenannten G Protein gekoppelten Rezeptoren (GPCRs) zur Herstellung eines Medikaments zur Behandlung und Prophylaxe von durch agonistische Autoantikörper (agAAK) bedingter Erkrankungen.

2. Verwendung gemäß Anspruch 1, wobei die GPCRs ausgewählt sind aus der Gruppe bestehend aus β1-R, β2-R, AT1-Rezeptor, a1-Rezeptor, muskarinerger R2, Endothelinrezeptor.

3. Verwendung nach einem der Ansprüche 2 und/oder 3, wobei die β1-Adrenorezeptor-Antagonisten insbesondere sind: Atenolol, Metoprolol, Bisoprolol, Propanolol, Sotalol, Pindolol, Carvedilol, Nebivolol zur Behandlung und Prophylaxe von chronischer Herzinsuffizienz, dilatativer Kardiomyopathie, Bluthochdruck, Tachykardien, koronarer Herzkrankheit, Migräne.

4. Verwendung nach Anspruch 2 und/oder 3, wobei die β2-Adrenorezeptor -Antagonisten insbesondere sind: Carvedilol, Timolol zur Behandlung und Prophylaxe von Glaukomen und antikörpervermittelten systemischen und lokalen Durchblutungsstörungen.

5. Verwendung nach Anspruch 2 und/oder 3, wobei die α-Adrenorezeptor - Antagonisten insbesondere sind: Phenoxybenzamin, Prazosin, Terazosin, Alfuzosin, Tamsulosin, zur Behandlung und Prophylaxe von Prostatahyperplasie, Hypertonie oder Tinnitus.

6. Verwendung nach Anspruch 2 und/oder 3, wobei die Angiotensin 1-Rezeptor - Antagonisten insbesondere sind: Valsartan, Losartan, Irbesartan, Candesartan, Eprosartan, Olmesartan, Telmisartan, zur Behandlung und Prophylaxe von Bluthochdruck, akutem Herzinfarkt, Nierenschädigung.

7. Verwendung nach Anspruch 2 und/oder 3, wobei die Endothelinrezeptor (ET1A)- Antagonisten insbesondere sind: Bosentan, Darusentan, zur Behandlung und Prophylaxe von pulmonaler arterieller Hypertonie, chronischer Herzinsuffizienz.
